Europäisches Patentamt

European Patent Office

Office européen des brevets

(11) Publication number: **0 684 470 A2**

# EUROPEAN PATENT APPLICATION

(21) Application number: **95109386.3**

(51) Int. Cl.⁶: **G01N 33/00, G01N 1/38**

(22) Date of filing: **23.07.91**

This application was filed on 16 - 06 - 1995 as a divisional application to the application mentioned under INID code 60.

(30) Priority: **25.07.90 JP 194946/90**

(43) Date of publication of application:
**29.11.95 Bulletin 95/48**

(60) Publication number of the earlier application in accordance with Art.76 EPC: **0 469 437**

(84) Designated Contracting States:
**DE GB**

(71) Applicant: **HITACHI, LTD.**
**6, Kanda Surugadai 4-chome**
**Chiyoda-ku,**
**Tokyo 101 (JP)**
Applicant: **HITACHI TOKYO ELECTRONICS CO., LTD.**
**3-2, Fujihashi 3-chome**
**Ome-shi**
**Tokyo (JP)**

(72) Inventor: **Irie, Takashi**
**Hitachi Daini Kyoshinryo, 1-3**
**Higashikoigakubo-3-chome,**
**Kokubunji-shi (JP)**
Inventor: **Mitsui, Yasuhiro**
**14-29, Nishifucho-4-chome**
**Fuchu-shi (JP)**
Inventor: **Mizokami, Kazuaki**
**Haitsu Namai 105,**
**2-26, Honcho-5-chome**
**Koganei-shi (JP)**

(74) Representative: **Patentanwälte Beetz - Timpe - Siegfried Schmitt-Fumian - Mayr**
**Steinsdorfstrasse 10**
**D-80538 München (DE)**

(54) **Method and apparatus for gas analysis.**

(57) The invention relates to a method and an apparatus for gas analysis, particularly for calibrating gas analysis devices with calibration gases containing very low concentrations of a liquid sample vapor at a ppb level. According to the invention, the method comprises the following steps:
- putting in the concentration of a sample gas in a calibration gas (18) into a computer system (40) to permit the system to calculate the flow rate (L3) of a zero gas (20) to be mixed with sample gas,
- supplying the zero gas (20) from a zero gas source (19) to a main tube (12),
- supplying the sample gas from a sample gas generating device (6) to the main tube (12) to mix the sample gas with the zero gas (20) in the main tube (12),
- controlling the flow rate of the zero gas (20) through flow control means (25) of the zero gas (20) by a set flow rate signal (43) of the calculated flow rate (L3) fed from the computer system (40) the thereby generate the calibration gas (18) to an analysis device (41), and
- feeding a data input-starting signal (46) from the computer system (40) to the analysis device (41), and feeding a data signal (47) from the analysis device (41) to the computer system (40).

Rank Xerox (UK) Business Services
(3.10/3.09/3.3.4)

# F I G. 3

This invention relates to a method and an apparatus for gas analysis and a method and an apparatus for the analysis of calibration gases containing a very low concentration of liquid sample vapor at a ppb (10-9) level or below, including the measures and means for preparing such gases or calibration gases, respectively.

Conventionally, mixing of a liquid sample vapor with a gas at a known concentration has been performed using an apparatus described in JIS-K0226 (Japanese Industrial Standard) where the sample is water. In this method, water is provided in a glass vessel equipped with a diffusion tube, and a stream of water vapor is discharged into a stream of gas. The water concentration in the gas is determined by measuring the amount of vaporization of the water (that is, the amount of reduction of the water) in the vessel by the use of a microbalance.

According to the JIS, the calibation gas having a water concentration of 2 to 20 ppm can be obtained with an error of 1 ppm. With respect to an error below this value, calibration gases cannot be prepared with this method, and JIS does not define any method of preparing such gas mixtures.

According to a dilution method and apparatus disclosed in JP-A-60-41733, a gas sample of a standard concentration is diluted with a highly purified gas the flow rate of which is controlled by a capillary tube, so as to obtain a low-concentration gas. In this example, a one-stage dilution is used; however, by combining a plurality of such stages, the dilution ratio may be increased. With this method, it is possible to obtain a low-concentration gas for non-adhesive components; however, in this apparatus, valves are provided in the flow passage of a standard concentration gas. The valves have a large inner surface area, and therefore in the case of a gas containing a low concentration of a highly-adhesive component (e.g. water), the component is adsorbed on the inner surface, which results in the serious problem that low-concentration gases at ppb levels cannot be obtained. In addition to the large inner surface area, the valves have a complicated structure, and this involves the further problem that the cleaning of the valves cannot be effected easily, so that producing gas mixtures comprising water at a ppb level is not easy.

Analytical Chemistry 49, 1977, pages 270 to 275, discloses an example in which the measurement of water is carried out using Atmospheric Pressure Ionization Mass Spectrometry (APIMS). In this example, a water bottle with a small aperture is provided in the gas stream, and by changing the diameter of the small aperture of the water bottle, the water evaporation rate is controlled so as to prepare calibration gases of a ppm level, and this calibration gas is analyzed by APIMS to thereby obtain a calibration curve in the ppm region.

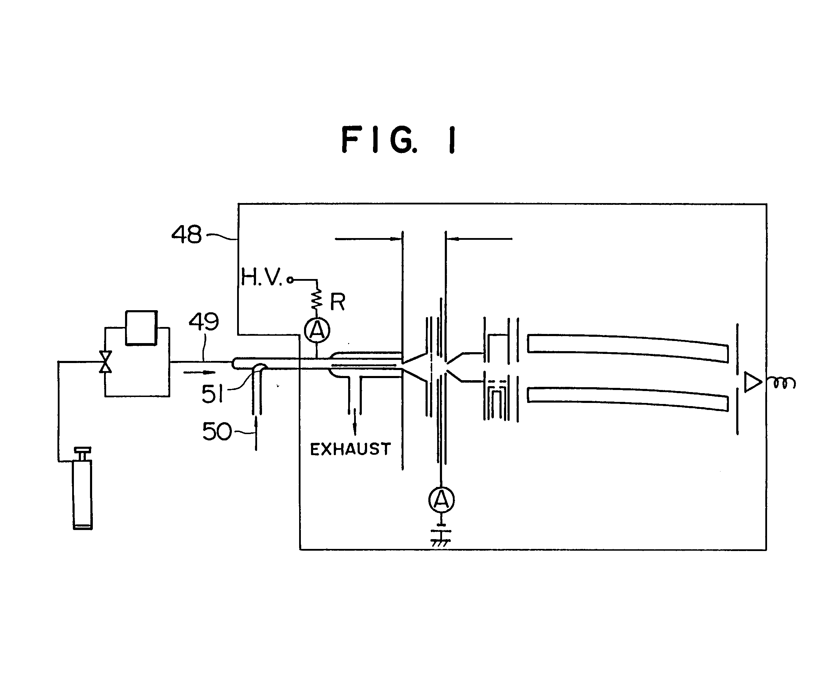

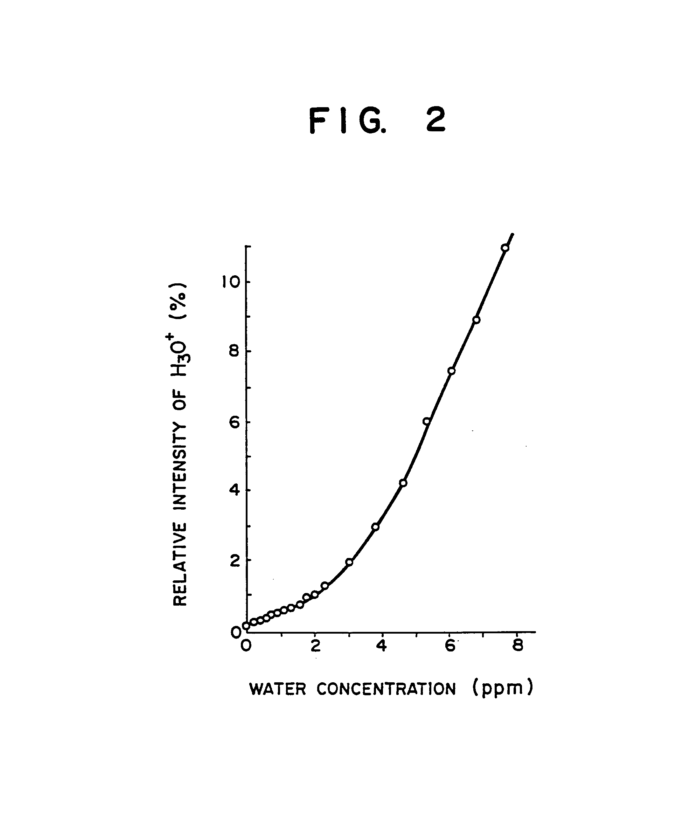

Fig. 1 shows a conventional apparatus for measuring a calibration curve of water using APIMS, and Fig. 2 shows an example of a measured calibration curve of water in oxygen gas measured with the apparatus of Fig. 1.

As shown in Fig. 2, even when only dry gas whose water concentration is zero is analyzed by APIMS 48, a water ion intensity corresponding to about 3 ppm of water is detected. Therefore, in the conventional apparatus shown in Fig. 1, considerable amounts of water are liberated at the sample gas introduction portion 49, 50, 51, so that calibration gases of a ppb level cannot be obtained at all.Therefore, a calibration curve of a ppb level cannot be obtained. It will be appreciated from this that in order to produce calibration gases of a ppb level and also to enable the measurement of the calibration curve in the ppb level region by APIMS, not only a suitable dilution method but also a reduction of the background water by appropriate structure and materials of the sample introduction system are indispensable.

One of the reasons why calibration curves can be obtained only at ppm levels in the conventional apparatus of Fig. 1 is that a high dilution ratio cannot easily be obtained because the evaporation rate of water is controlled by changing the diameter of the aperture of the water bottle, and thus, the control of the gas production at a low concentration of a ppb level is difficult. However, if the problem is only with the dilution method, the standard gas containing a controlled concentration of the liquid sample vapor at a ppb level or below must be thoretically prepared by combining the water producing device of JIS-K0226 with a multi-stage dilution system (for example, a two-stage dilution system) constituted by a plurality of combined dilution systems according to JP-A-60-41733, that is, by preparing the calibration gas containing the liquid sample vapor of 2 to 20 ppm level and then by diluting it by the two-stage dilution system. However, with respect to such a liquid sample as water, (1) it takes a long time to establish an equilibrium between the adsorption and desorption of the sample relative to the inner surface of the tube because the effect of adsorption on the inner surface of the tuber is great. As a result, the accuracy of the concentration is lowered, and the measurement time is prolonged; (2) since the background produced from the tube is large, the dilution ratio does not accurately reflect the concentration at a ppb level; (3) the coexisting components which quench the sample ions are produced much to a considerable extent. Because of these problems, with respect to the two-stage gas dilution system, the area of the inner surface and the dead zone must be reduced by a compact construction thereof, and also the adsorption effects must be reduced by treating the inner surface of the tube and by heating the tube to elevated temperatures. In the above prior art, however, the gas inlet system is assembled with valves and flow controllers, or a tube not subjected to an inner surface treatment is used, and no consideration has been given to these problems. Therefore, using the apparatus shown in Fig. 1, it has been impossible to prepare or analyze calibration gases of a ppb level because the water background is high, as shown in Fig. 2.

It is the object of the present invention to provide a method and an apparatus for the quantitative analysis of gases and a method and an apparatus for the quantitative analysis of calibration gases, particularly those containing a very low concentration of a liquid sample vapor at a ppb level or below, and to provide measures and means for preparing such gases, in particular calibration gases.

It is a further object to provide a method and an apparatus for the quantitative analysis of gases and particularly of calibration gases wherein a computer system is used both for controlling the preparation of the gas to be analyzed, in particular of a calibration gas, and for controlling the analysis device.

It is a further object of this invention to provide a method and an apparatus for the gas analysis by which a calibration gas, containing a controlled, very low concentration of a highly-adhesive component, such as water, at a ppb level or below, can be rapidly prepared with a precise concentration.

The above object is achieved according to the independent claims. The dependent claims relate to prefered embodiments of the concept of the present invention.

The method for gas analysis of the present invention comprises the following steps:
- putting in the concentration of a sample gas in a calibration gas into a computer system to permit the system to calculate the flow rate of a zero gas to be mixed with sample gas,
- supplying the zero gas from a zero gas source to a main tube,
- supplying the sample gas from a sample gas generating device to the main tube to mix the sample gas with the zero gas in the main tube,
- controlling the flow rate of the zero gas through flow control means of the zero gas by a set flow rate signal of the calculated flow rate fed from the computer system to thereby generate the calibration gas to an analysis device, and
- feeding a data input-starting signal from the computer system to the analysis device, and feeding a data signal from the analysis device to the computer system.

The analysis device preferably comprises an atmospheric pressure ionization mass spectrometer.

The computer system perferably serves to perform processing of the data signals.

According to a prefered embodiment, the computer system receives a flow rate data signal fed from the flow control means to control the flow control means in feeding the set flow rate signal to the computer system.

In accordance with another prefered embodiment,
- a confirmation signal is sent from a controlling unit to the computer system, the controlling unit receiving flow rate-setting signals from the computer system and putting out the set flow rate signals and receiving the flow rate data signals from the flow control means, and
- the computer system sends a data input-starting signal to the analysis device and then receives data signals from the analysis device and processes the data signals to generate the analysis data, preferably in the form of spectra.

According to another prefered embodiment, the calibration gas is prepared by the following steps:
- supplying a first zero gas from a first zero gas source with a controlled flow rate,
- supplying a sample gas from a sample gas source with a controlled flow rate,
- mixing the sample gas with the first zero gas in a mixing chamber thus forming a first standard gas containing the sample gas in a predetermined concentration,
- discarding a part of the first standard gas at a first branch with a controlled flow rate, and
- mixing the remaining first standard gas at a second branch downstream of the first branch with a second zero gas from a second zero gas source with a controlled flow rate to obtain a diluted, second standard gas,

wherein the sample gas is supplied by vaporizing a liquid sample as sample gas source with a controlled evaporation rate,

and wherein the diluted standard gases are prevented from coming into contact with any parts such as valves and flow controllers,

to obtain a calibration gas containing the sample gas in a very low concentration.

According to another prefered embodiment, a two-stage gas dilution is applied wherein a part of the second standard gas is discarded at a third branch with a predetermined flow rate, and

the remaining diluted standard gas is mixed at a fourth branch downstream of the third branch with a third zero gas from a third zero gas source with a controlled flow rate.

EP 0 684 470 A2

Such a two-step gas dilution method per se is subject-matter of the parent patent application EP 91112323.0-2204 (EP 469 437).

Further prefered embodiments are characterized by one or a combination of the following measures:
- the amount of vaporized liquid sample is measured with a microbalance or with an analysis device;
- the first and second and optionally the third zero gases are provided from one gas source line;
- the zero gas is purified directly before mixing with the sample gas or standard gas; respectively;
- the main tube is maintained at a temperature 20 to 50 °C higher than the boiling point of the liquid sample.

The apparatus for gas analysis of the present invention comprises:
- a main tube connected at its one end to an analysis device for forming a calibration gas from a zero gas and a sample gas,
- zero gas source means for supplying zero gas to the main tube, the gas source means comprising flow control means for controlling the flow rate of the zero gas,
- a sample gas generating device for supplying the sample gas to the main tube, and
- a computer system for calculating the flow rate of the zero gas for a concentration of the sample gas in the calibration gas, which concentration is put in into the computer system, feeding a set flow rate signal of the calculated flow rate to the flow control means to control the flow rate, feeding a data input-starting signal to the analysis device and receiving a data signal from the analysis device.

The analysis device preferably comprises an atmospheric pressure ionization mass spectrometer.

According to a prefered embodiment, the computer system serves to perform processing of the data signals.

In the apparatus according to the present invention, the computer system is preferably connected to a controlling unit which receives a flow-rate setting signal from the computer system, feeds a set flow rate signal to the flow control means, receives a flow rate control signal from the flow control means, controls the flow control means so as to confirm the flow rate of the flow control means, and feeds a confirmation signal to the computer system.

According to a further prefered embodiment, the computer system receives a flow rate data signal fed from the flow control means to control the flow control means through the set flow rate signals.

In accordance with a further prefered embodiment of the apparatus according to the present invention, the device for preparing the calibration gas comprises:
- a first and a second zero gas source sor supplying a first zero gas and a second zero gas, comprising means for controlling the flow rates of the first or second zero gases, respectively,
- a sample gas source for supplying a sample gas, comprising means for controlling the flow rate of the sample gas,
- a mixing chamber for mixing the sample gas with the first zero gas, thus forming a first standard gas, and
- a main tube connected with the mixing chamber and having
(a) a first branch comprising controlling means for discarding a part of the first standard gas with a controlled flow rate,
(b) a second branch provided downstream of the first branch, at which the remaining first standard gas is mixed with the second zero gas with a controlled flow rate for obtaining a diluted, second standard gas,
wherein the mixing chamber contains a sample vessel in which a liquid sample may be vaporized for supplying the sample gas, and the means for controlling the flow rate of the sample gas are means for controlling the evaporation rate of the liquid sample from the sample vessel,
the main tube not being provided with any parts such as valves and flow controllers.

According to still another prefered embodiment, a two-stage gas dilution system is implemented wherein the main tube is provided with
(a) a third branch comprising controlling means for discarding a part of the diluted second standard gas with a predetermined flow rate, and
(b) a fourth branch provided downstream of the third branch, at which the remaining diluted second standard gas is mixed with a third zero gas supplied from a third zero gas source comprising means for controlling the flow rate of the third zero gas.

Such a two-stage gas dilution system per se forms subject-matter of the parent patent application EP 91112323.0-2204 (EP 469 437).

Other prefered embodiments of the apparatus according to the present invention are characterized by one or a combination of the following features:

5

- The sample vessel comprises a variable capillary aperture, preferably of 0.1 to 5 mm, for adjusting the evaporation rate of the liquid sample;
- the sample vessel comprises a planar or curved upper surface which has no projection;
- the tubes are made of electropolished stainless steel or glass, the steel tubes being connected together entirely by welding;
- control means are provided to control the flow rates of the flow controlling means and the concentration of the calibration gas automatically;
- the mixing chamber is provided with temperature control means for controlling the evaporation rate of the liquid sample from the sample vessel;
- adsorption reduction means are provided at the main tube for reducing the gas adsorption, preferably means for maintaining the main tube at a temperature 20 to 50 °C higher than the boiling point of the liquid sample;
- impurity removal devices are provided in the zero gas supply lines directly before the mixing chamber or at the branches, respectively.

The invention further encompasses the application of the method and of the apparatus as defined above to the analysis of impurities present in a gas in a very low concentration.

According to a prefered embodiment of the present invention, a predetermined quantity of standard gas is produced from a gas source, and then a predetermined proportion of the gas is exhausted at least once, and then a predetermined amount of dilution gas is supplied at least once, thereby obtaining a calibration gas containing a very low concentration of liquid sample vapor. In the present invention, gas prepared beforehand, for example, a cylinder gas, can be used as the standard gas.

Structure and material of a device for preparing a standard gas containing a known concentration of a vaporized sample at a ppm level, as well as the structure and material of a two-stage gas dilution system, have been studied, and the following contrivances have been made: (1) Electropolished stainless steel tubes or glass tubes are used and connected together entirely by welding, so as to achieve a compact construction of the apparatus, thereby reducing the area of the inner surface and also minimizing the dead zone where the gas stream stagnates. (2) Any parts (such as valves and flow controllers) which would be sources of background, and coexisting components which could quench sample ions are not comprised in the main tube. (3) The gas is controlled in pressure and flow rate by a gas regulator and a flow controller, and then the impurities are removed from the gas by an impurity removal device, and then the gas is introduced into the device for preparing the standard gas containing a known concentration of the vaporized sample at a ppm level or the two-stage gas dilution system. (4) The main tube of the two-stage gas dilution system is maintained at a temperature 20 to 50 °C higher than the boiling point of the liquid sample so as to efficiently establish an equilibrium between the adsorption and desorption of the sample relative to the inner surface of the tube.

In the apparatus of the present invention, the calibration gas containing a very low concentration of the liquid sample at a ppb level is prepared according to the following procedure. First, the standard gas containing a high concentration (ppm level) of the vaporized sample is prepared in the following manner.

The gas is controlled in pressure by the gas regulator, and is controlled in flow rate by the flow controller, and then is introduced into a sample chamber. A sample bottle (vessel) which has a small aperture and contains the liquid sample is accommodated within the sample chamber. The vapor of the liquid sample is discharged into the stream of the gas through this aperture, and is mixed therewith. The upper surface of the sample bottle with the small aperture is a planar surface or a curved surface having no projection, and the small aperture is formed in this surface. In JIS-K0226, it is defined that a sample bottle should have a projection like a needle for vaporized sample diffusion, in which case the sample liquid often stagnates midway in this needle-like diffusion projection, so that the amount of vaporization is rendered unstable. For this reason, such a projection is eliminated in the present invention. Therefore, the sample liquid will not stagnate midway in the diffusion projection, and the standard gas containing a high concentration (ppm level) of the vaporized sample can be obtained in a stable manner.

By providing the impurity removal device (e.g. a molecular sieve trap) at the upstream side of the sample chamber, adhesive impurities, such as water and organic components, are removed from the gas. Therefore, the concentration accuracy of the standard gas containing the high concentration of the vaporized sample is not lowered, and also coexisting components which quench sample ions will not be introduced into the gas. The flow controller serves to keep constant the flow rate (L1 ($\ell$/min)) of the gas flowing through the sample chamber, and temperature control means provided outside the sample chamber serve to keep the temperature of the sample constant. Therefore, the high-concentration vaporized sample-containing standard gas which is always kept at a constant concentration and a constant flow rate is supplied to the two-stage gas dilution system.

EP 0 684 470 A2

For calibrating the concentration of the sample in the standard gas containing the high concentration of the vaporized sample, the amount of vaporization (i.e., the vaporized quantity) of the liquid sample in the sample bottle when the gas continues to flow at a constant rate for a long period of time is measured by a microbalance.

This calibration method is the same as that described in JIS-K0226. More specifically, the concentration C (ppm) of the standard gas containing the high concentration of the vaporized sample is expressed by the following formula:

$$C = \frac{22.4 \times \frac{R}{M}}{L1 \times \frac{273}{273 + t} \times \frac{P}{760}} \quad ,$$

where R ($\mu$g/min) represents the vaporized quantity of the liquid sample per unit time, M represents the molecular weight of the liquid sample, t ($^\circ$C) represents the temperature, P (torr) represents the gas pressure, and L1 ($\ell$/min) represents the gas flow rate.

If, for example, water is used as liquid sample, and the diameter of the aperture of the sample bottle is 1 mm, and when the gas is flowing at a flow rate of 1 $\ell$/min at 20 $^\circ$C and 760 torr (101 325 Pa) for 10 h, the vaporized quantity is about 14 mg, and therefore the concentration of the standard gas containing the high concentration of the vaporized sample is about 30 ppm. In the case of using dioctyl phthalate (DOP) as liquid sample at 170 $^\circ$C, and for a diameter of the aperture of the sample bottle of 5 mm, and a gas flow rate of 0.3 $\ell$/min for 6 h, the vaporized quantity is about 2 mg, and therefore the concentration of the standard gas containing the high concentration of the vaporized sample is about 1.7 ppm.

The diameter of the aperture of the sample bottle is an important factor in determining the amount of vaporization of the sample vapor into the gas, and needs to be changed depending on the vapor pressure of the sample. More specifically, the aperture diameter must be made small with respect to a sample having a high vapor pressure, and must be made large with respect to a sample having a low vapor pressure so as to keep the concentration of the sample in the standard gas (which contains the high concentration of the vaporized sample) to a level of 1 ppm to several tens of ppm. Referring to the reason for this, if the concentration is too high, the gas cannot be diluted from the maximum dilution ratio of the two-stage gas dilution system to a ppb level. On the other hand, if the concentration is too low, the vaporized quantity of the liquid sample is small, and even by the use of the microbalance, the precision of the weighing (and hence the precision of the calibration) is lowered because of weight variations caused by adsorption of water in the air on the sample bottle and the sample itself. In this case, in order to obtain a sufficient vaporized quantity, a long period of time is required for the calibration of the concentration. Thus, these disadvantages are encountered.

Actually, by changing the temperature of the sample by the temperature control means provided outside the sample chamber, the vapor pressure can be controlled, and therefore the sample concentration is not controlled only by the aperture diameter. However, as the set temperature deviates from the ordinary (room) temperature (around 20 $^\circ$C), the temperature control means cannot maintain a uniform gas and sample temperature, which would become complicated. Therefore, also the control of the aperture diameter is important. When water is used as the liquid sample, a standard gas having a sample concentration of about 1 to about 30 ppm can be obtained by setting the aperture diameter to 0.1 to 1 mm. In the case of dioctyl phthalate, a standard gas having a sample concentration of about 1 to about 10 ppm can be obtained with an aperture diameter of 5 mm.

Reference is now made to a method in which the high-concentration vaporized sample-containing standard gas prepared according to the above procedure is diluted by the two-stage gas dilution system to prepare the calibration gas containing a very low concentration of liquid sample vapor at a ppb level or below.

The standard gas containing the high concentration of the vaporized sample is introduced into the main tube, and then part of this standard gas is exhausted at a first branch (flow rate: L2 ($\ell$/min)). The flow rate of the exhausted gas is controlled by a flow controller provided at the first branch. The reason for the exhausting of the gas at the first branch is that assuming that any part of the gas is not exhausted (L2 = 0), in order to obtain a high dilution ratio (for example, 100 times), L3 must be about 100 times as large as L1. Usually, in order to achieve stability of the concentration of the standard gas containing the high concentration of the vaporized sample, L1 must be about 1 $\ell$/min, and therefore L3 must be about 100 $\ell$/min. To use such a large quantity of gas is uneconomical. Actually, if L1 and L2 are 1 $\ell$/min and 0.99

7

ℓ/min, respectively, then L3 = 1 ℓ/min is only required for obtaining a 100 times dilution ratio. This is economical. The remaining flow rate (L1 - L2) of the standard gas containing the high concentration of vaporized sample is diluted by gas (flow rate: L3 (ℓ/min)) introduced from a second branch (first-stage dilution). The gas introduced from the second branch is controlled in pressure and flow rate by a gas regulator and a flow controller, and then impurities are removed from this gas by an impurity removal device. Therefore, the background vapor and coexisting components which quench sample ions are not introduced into the gas. With this first-stage dilution, there is obtained a gas the flow rate of which is represented by L1 - L2 + L3 (ℓ/min), and the concentration of the sample in the gas is represented by the following formula:

$$C \times \frac{L1 - L2}{L1 - L2 + L3} \ (\text{ppm})$$

where C represents the concentration of the sample in the standard gas containing the high concentration of vaporized sample.

Part of the gas (flow rate: L1 - L2 + L3) obtained by the first-stage dilution is exhausted from a third branch (flow rate: L4 (ℓ/min)). The gas is exhausted from the third branch for the same reason described above for the exhausting of the gas at the first branch.

The gas of the remaining flow rate (L1 - L2 + L3 - L4) is again diluted by gas (flow rate: L5 (ℓ/min)) introduced from a fourth branch (second-stage dilution). Like the gas introduced from the second branch, the gas introduced from the fourth branch is controlled in pressure and flow rate by a gas regulator and a flow controller, and then impurities are removed from this gas by an impurity removal device. With this two-stage dilution, there is obtained the calibration gas having a very low concentration of vaporized sample where the flow rate is represented by L1 - L2 + L3 - L4 + L5, and the concentration is represented by C [- (L1 - L2)/(L1 - L2 + L3)][(L1 - L2 + L3 - L4)/(L1 - L2 + L3 - L4 + L5)]. Here, C represents the concentration of the sample in the standard gas containing the high concentration of the vaporized sample.

In the above procedure, the gases, introduced respectively from the device for preparing the standard gas containing a known concentration of the vaporized sample and the second and fourth branches of the two-stage gas dilution system, are of the same kind.

If mass flow controllers are used as flow controllers for controlling the flow rates L1 to L5, the mass flow rates can be directly measured, and therefore errors in the measurement of L1 to L5 due to variations of the gas volume caused by the gas pressure and the gas temperature can be avoided, thereby enabling a highly precise dilution.

In order that in the very low concentration range of a ppb level, the actual concentration can immediately correspond correctly to the concentrations determined by the above flow rate controls (L2 to L5), it is important to reduce the generation of the background at the main tube and also to rapidly establish an equilibrium between the adsorption and desorption of the sample relative to the inner surface of the tube. In the present invention, the main tube is constituted by welding electropolished stainless steel tubes or glass tubes, and the short straight sections of the main tube are connected together so as to minimize the area of the inner surface thereof. Those parts (such as joints, valves and flow controllers) which would form dead zones and sources of impurities are eliminated from the main tube. Further, by the use of the temperature control means, the temperature of the main tube is maintained at a temperature 20 to 50 °C higher than the boiling temperature of the liquid sample. With this arrangement, a rapid establishment of equilibrium between adsorption and desorption as well as a reduction of the background can be achieved.

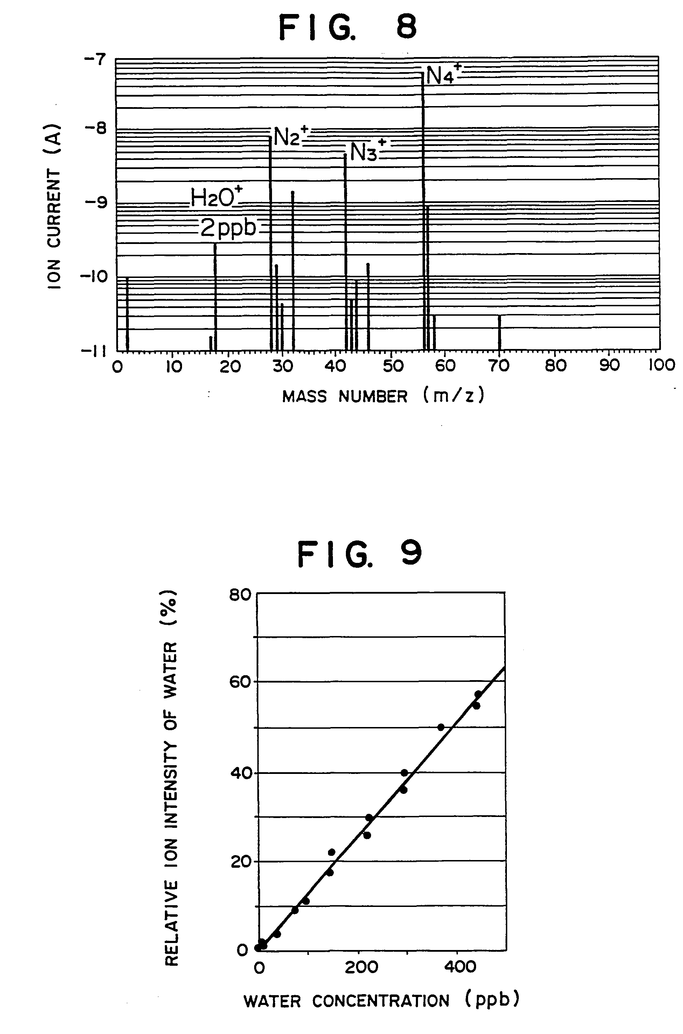

As one example, using the above-explained system, the background for the water in nitrogen gas can be reduced to 2 ppb by shortening the main tube to a length of 30 cm. This has been confirmed by APIMS, and results thereof are shown in Fig. 8. At this time, the time required for establishing equilibrium between adsorption and desorption was within about several tens of seconds.

As described above, by controlling the flow rates L2 to L5 at the two-stage dilution portion, the calibration gas containing a controlled known concentration of the liquid sample vapor in a very low concentration range of a ppb level can be prepared.

A one-stage dilution may be used as the dilution method. In this case, the flow passages of L4 and L5 are omitted. However, in this case, since the flow rate L1 - L2 is very small, high precision is required for the flow controllers (generally, the error must be kept to below 0.1 ml/min relative to the flow rate of 1 ℓ/min).

In the following, the invention will be further explained with reference to the drawings which relate to preferred embodiments of the invention.

Fig. 3 is a schematic view of one preferred embodiment of an apparatus of the present invention, in which a one-stage gas dilution system is used;

Figs. 4, 5 and 6 show two-stage gas dilution systems according to the parent patent application EP 91112323.0-2204 (EP 469 437).

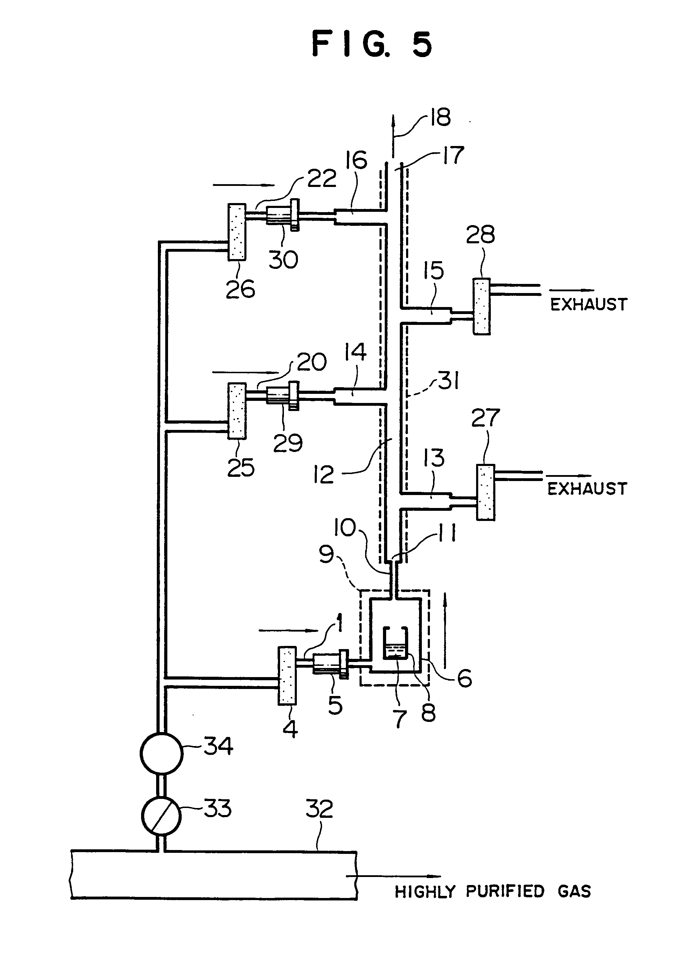

The system of Fig. 5 may be used in facilities such as clean rooms, in which a line gas is used.

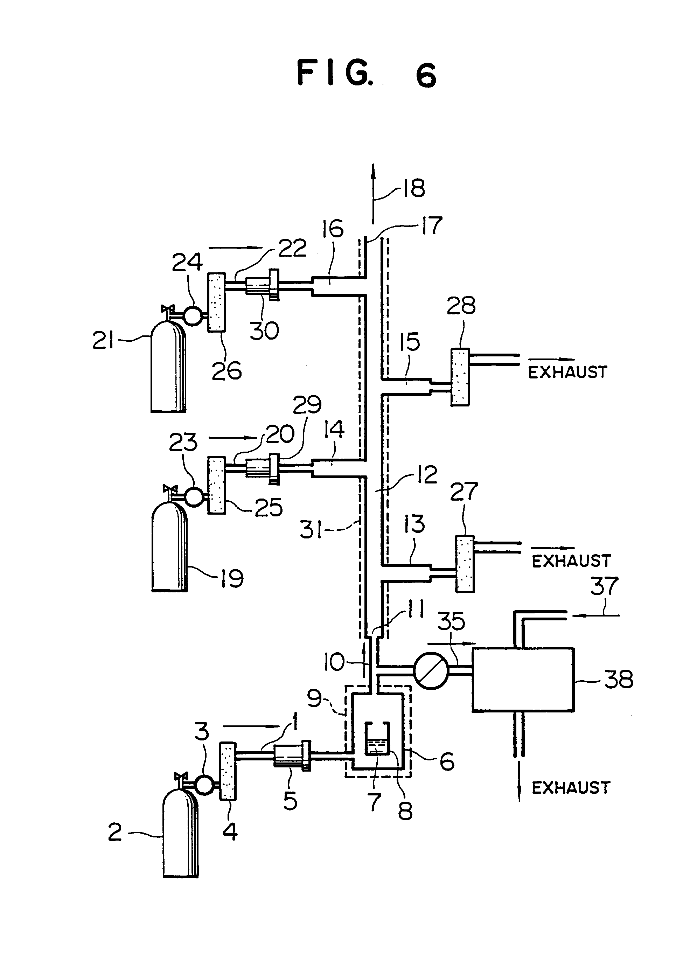

The system of Fig. 6 comprises a gas chromatograph for calibrating the concentration of a standard gas containing a high concentration of a sample.

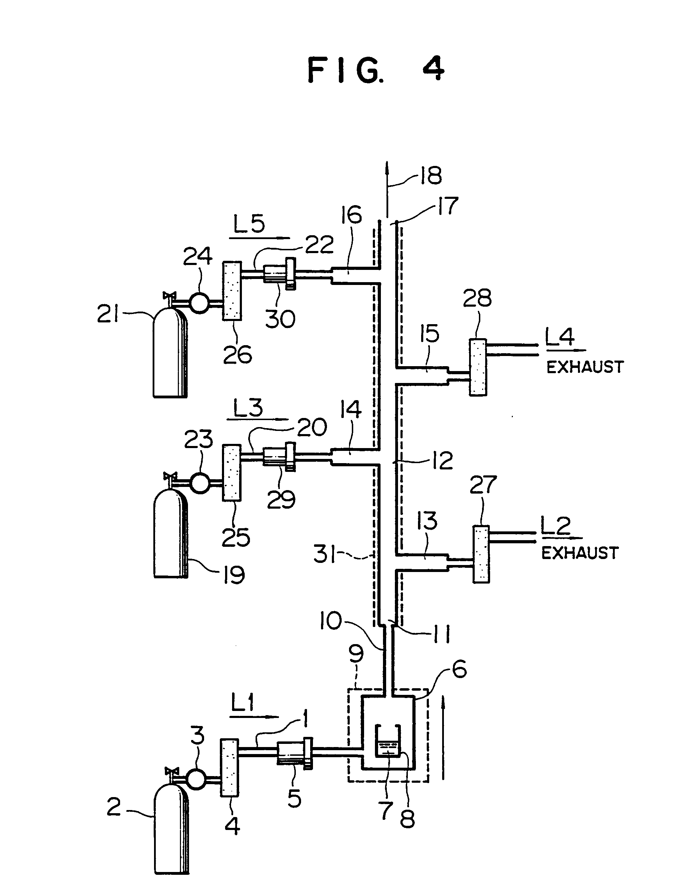

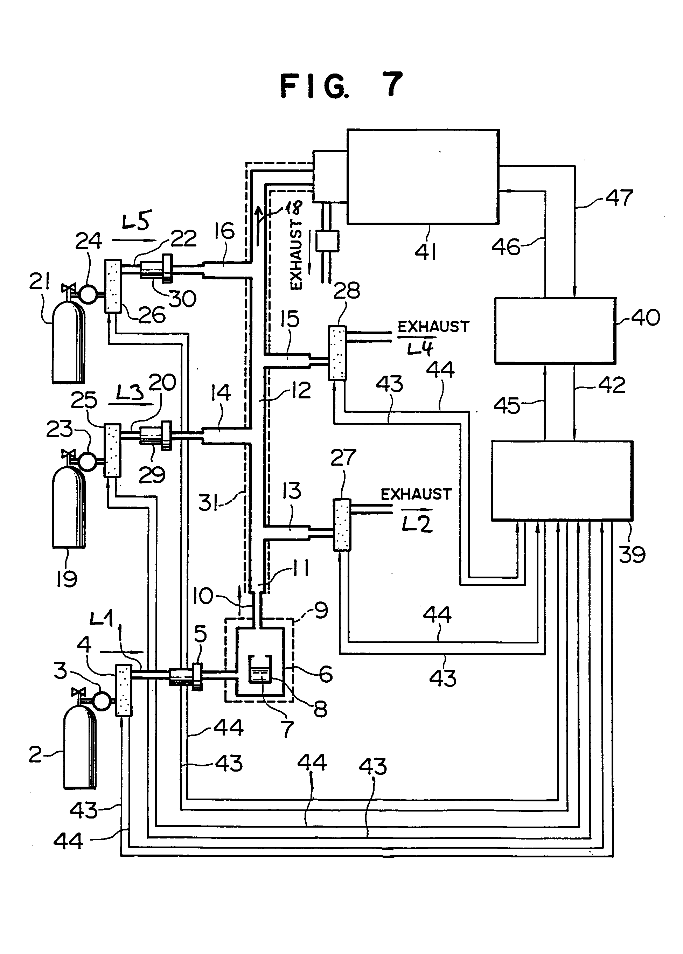

Fig. 7 is a schematic view of a further prefered embodiment of the apparatus of the present invention wherein a two-stage gas dilution system according to Fig. 4 is used for calibrating the analysis device (which is, e.g., an APIMS spectrometer or a dew-point hygrometer) for analyzing a very low concentration of impurities in a gas, and a calibration curve is automatically produced.

Fig. 8 is a diagram showing data of the background level of water produced from the apparatus of Fig. 7 which data are measured by APIMS.

Fig. 9 is a graph showing results of measurement of a calibration curve by APIMS for water, using calibration nitrogen gas containing a very low concentration of water obtained with the apparatus of Fig. 6 using the two-stage gas dilution system of Fig. 4.

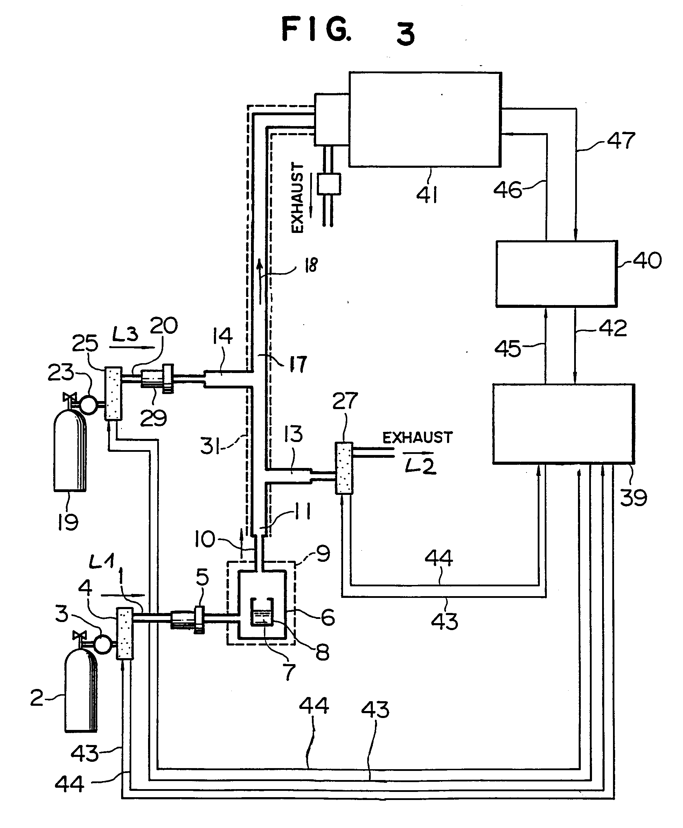

A preferred embodiment of the present invention will now be described with reference to Fig. 3.

The apparatus of the invention comprises a device for preparing calibration gas containing a known concentration of a vaporized sample, using a one-stage gas dilution system. In this device, first zero gas 1 is fed from a gas cylinder 2, and is controlled in pressure and flow rate by a gas regulator 3 and a flow controller 4. Adhesive impurities, such as water and organic components, are removed from the gas 1 by an impurity removal device 5, such as a molecular sieve trap, and then the gas 1 is introduced into a mixing chamber 6 serving as sample vapor-generating device. The impurities to be removed by the impurity removal device 5 include those originally contained in the gas 1 and those components originating from the gas regulator and the flow controller. In the case where the gas regulator and the flow controller cannot be sufficiently cleaned by high-temperature heating and the passing of gas therethrough, the use of the impurity removal device 5 is indispensable for the preparation of the calibration gas containing a sample whose concentration is at a ppb level or below, because such impurities adversely affect the preparation of this calibration gas.

A sample vessel 8 containing a liquid sample 7 is accommodated within the mixing chamber 6. The sample vessel 8 has an aperture with a diameter of 0.1 to 5 mm, and the vapor of the sample 7 is discharged into a stream of the gas 1 through this aperture. The whole mixing chamber 6 is maintained at a predetermined temperature by temperature control means 9, and the pressure of the vapor of the liquid sample 7 (and hence the amount of discharge of the sample vapor into the stream of the gas 1) is kept constant. The concentration of the sample 7 in the gas 1 passed past the mixing chamber 6 is calibrated by measuring the amount of vaporization of the sample 7 by a microbalance when the gas 1 continues to flow at a constant rate for a long period of time (about 10 h).

As one example, water is used as the sample 7, and the temperature of the mixing chamber 6 is maintained at 20 °C, and the flow rate of the gas 1 is maintained at 1 ℓ/min. In this case, when the diameter of the aperture of the sample vessel 8 is 1 mm, the water concentration of the gas 10 is about 30 ppm. As another example, the dioctyl phthalate (DOP) is used as the sample 7, and the temperature of the mixing chamber 6 is maintained at 170 °C, and the flow rate of the gas 1 is maintained at 0.3 ℓ/min. In this case, when the diameter of the aperture of the sample bottle 8 is 5 mm, the DOP concentration of the gas 10 is about 2 ppm.

The gas 10 thus containing a predetermined concentration of the sample 7 is introduced into a main tube (vaporized sample supply passage) 12 of the one-stage gas dilution system via a gas inlet 11.

The one-stage gas dilution system comprises the main tube 12, two branches 13 and 14, the gas inlet 11, and a gas outlet 17. In this gas dilution system, the gas 10 containing the predetermined concentration of the sample 7 is diluted according to the following procedure to be converted into a calibration gas 18 containing a given concentration of the vaporized sample 7.

The gas 10 of a constant flow rate L1 containing the predetermined concentration of the sample 7 is introduced into the main tube 12 via the gas inlet 11, and part (flow rate: L2) of the gas 10 is first exhausted at the first branch 13. This flow rate control is effected by a flow controller 27. Then, the gas 10 is diluted at the second branch 14 by gas 20 (flow rate: L3) supplied from a gas cylinder 19 (first-stage dilution). The calibration gas 18 is discharged from the gas outlet 17. The zero gas 20 is of the same kind as that of the zero gas 1, and is controlled in pressure and flow rate by the gas regulator 23 and the flow controller 25.

For the same reasons described above for the gas 1, adhesive impurities are sufficiently removed from the gas 20 by the impurity removal device 29.

Incidentally, if the gases 1 and 20 have a sufficiently high purity, and if the amounts of the impurities released from the gas regulators 3 and 23 and the flow controllers 4 and 25 are sufficiently small, the impurity removal devices 5 and 29 may be omitted.

The concentration of the sample in the gas 18 is represented by C [(L1 - L2)/(L1 - L2 + L3)] where C represents the concentration of the sample in the standard gas 10 of the constant flow rate L1. By changing the flow rates L2 and L3 respectively by the flow controllers 27 and 25, there can be obtained the calibration gas 18 containing a controlled, very low concentration of the sample.

The apparatus shown in Fig. 3 further comprises an analysis device (e.g. an APIMS spectrometer) for analyzing a very low concentration of impurities in gas for calibration purposes.

The gas outlet 17 is connected directly to the analysis device 41, and the calibration gas 18 is introduced into the analysis device 41, so that the spectra can be observed. By controlling the flow controllers 25 and 27, the concentration is set to a desired value, and the ion intensity of the sample is measured with respect to each of the concentrations, and the calibration curve representative of the relation between the concentration and the ion intensity can be measured.

In this embodiment, the flow controllers 4, 25 and 27 are controlled by a flow controller-controlling unit 39 and a computer system 40. More specifically, when the set concentrations are input into the computer system 40, the set values of the flow rates L1 to L3 (here, L1 is constant) are automatically calculated, and a flow rate-setting data signal 42 is fed to the controlling unit 39. The controlling unit 39 sends set flow rate signals 43 to the respective flow controllers, and reads flow rate data signals 44, fed respectively from the flow controllers, so as to confirm that the flow rates have been properly set respectively in the flow controllers. Thereafter, the controlling unit 39 sends a confirmation signal 45 to the computer system 40. Upon receipt of this confirmation signal 45, the computer system 40 sends a data input-starting signal 46 to the analysis device 41, and then receives a data signal 47 from the analysis device 41 and processes this data signal to prepare the spectra.

In this embodiment, there is the great advantage that the calibration curve of the analysis device for analyzing the concentration of impurities in the gas containing the liquid sample 7 can be automatically produced. And besides, the computer system 40 can be used not only to control the flow controllers but also to control the analysis device for analyzing the concentration of impurities in the gas, and therefore the cost can be reduced.

In Figs. 4-6, two-stage gas dilution systems according to the parent application EP 91112323.0-2204 (EP 469 437) are shown which can be used advantageously in the present invention.

The gas dilution system of Fig. 4 comprises a device for preparing standard gas containing a known concentration of a vaporized sample at ppm level (this standard gas will be hereinafter referred to as "high-concentration standard gas"), and represents a two-stage gas dilution system. The first stage of this system is the same as that used in the apparatus of Fig. 3. In the device for preparing the high-concentration standard gas, first zero gas 1 is fed from a gas cylinder 2, and is controlled in pressure and flow rate by the gas regulator 3 and the flow controller 4. Adhesive impurities, such as water and organic components, are removed from the gas 1 by the impurity removal device 5, such as a molecular sieve trap, and then the gas 1 is introduced into the mixing chamber 6 serving as sample vapor-generating device. The impurites to be removed by the impurity removal device 5 include those originally contained in the gas 1 and those components originating from the gas regulator and the flow controller. In the case where the gas regulator and the flow controller cannot be sufficiently cleaned by high-temperature heating and the passing of gas therethrough, the use of the impurity removal device 5 is indispensable for the preparation of the calibration gas containing a sample whose concentration is at a ppb level or below, because such impurities adversely affect the preparation of this calibration gas.

The sample vessel 8 containing a liquid sample 7 is accommodated within the mixing chamber 6. The sample vessel 8 has an aperture with a diameter of 0.1 to 5 mm, and the vapor of the sample 7 is discharged into the stream of the gas 1 through this aperture. The whole mixing chamber 6 is maintained at a predetermined temperature by the temperature control means 9, and the pressure of the vapor of the liquid sample 7 (and hence the amount of discharge of the sample vapor into the stream of the gas 1) is kept constant. The concentration of the sample 7 in the gas 1 (hereinafter referred to as "gas 10" (first standard gas)) passed past the mixing chamber 6 is calibrated by measuring the amount of vaporization of the sample 7 by a microbalance when the gas 1 continues to flow at a constant rate for a long period of time (about 10 h).

As one example, water is used as the sample 7, and the temperature of the mixing chamber 6 is maintained at 20 °C, and the flow rate of the gas 1 is maintained at 1 $\ell$/min. In this case, when the diamater of the aperture of the sample vessel 8 is 1 mm, the water concentration of the gas 10 is about 30 ppm. As another example, dioctyl phthalate (DOP) is used as the sample 7, and the temperature of the mixing chamber 6 is maintained at 170 °C, and the flow rate of the gas 1 is maintained at 0.3 $\ell$/min. In this case, when the diameter of the aperture of the sample bottle 8 is 5 mm, the DOP concentration of the gas 10 is about 2 ppm.

The gas 10 thus containing a predetermined concentration of the sample 7 is introduced into the main tube 12 of the two-stage gas dilution system via the gas inlet 11.

The two-stage gas dilution system comprises the main tube 12, four branches 13, 14, 15 and 16, the gas inlet 11, and a gas outlet 17. In this two-stage gas dilution system, the gas 10 containing the predetermined concentration of the sample 7 is diluted according to the following procedure to be converted into a calibration gas 18 containing a very low concentration (ppb level) of the vaporized sample 7. (This calibration gas 18 will hereinafter be referred to as "the very low-concentration calibration gas 18").

The gas 10 of a constant flow rate L1 containing the predetermined concentration of the sample 7 is introduced into the main tube 12 via the gas inlet 11, and part (flow rate: L2) of the gas 10 is first exhausted at the first branch 13. This flow rate control is effected by a flow controller 27. Then, the gas 10 is diluted at the second branch 14 by gas 20 (flow rate: L3) supplied from a gas cylinder 19 (first-stage dilution). Part (flow rate: L4) of this first-stage diluted gas is exhausted at the third branch 15. This flow rate control is effected by a flow controller 28. The diluted gas is further diluted at the fourth branch 16 by gas 22 (flow rate: L5) supplied from a gas cylinder 21 (second-stage dilution) to provide the very low-concentration calibration gas 18 which is discharged from the gas outlet 17. The zero gases 20 and 22 are of the same kind as that of the zero gas 1, and are respectively controlled in pressure and flow rate by gas regulators 23 and 24 and flow controllers 25 and 26. For the same reasons described above for the gas 1, adhesive impurities are sufficiently removed from the gases 20 and 22 by impurity removal devices 29 and 30.

Incidentally, if the gases 1, 20 and 22 have a sufficiently high purity, and if the amounts of the impurities released from the gas regulators 3, 23 and 24 and the flow controllers 4, 25 and 26 are sufficiently small, the impurity removal devices 5, 29 and 30 may be omitted.

The concentration of the sample 7 in the very low-concentration calibration gas 18 prepared according to the above procedure is represented by C [(L1 - L2)/(L1 - L2 + L3)][(L1 - L2 + L3 - L4)/(L1 - L2 + L3 - L4 + L5)]. Here, C represents the concentration of the sample in the standard gas 10 of the constant flow rate L1. By changing the flow rates L2, L3, L4 and L5 respectively by the flow controllers 27, 25, 28 and 26, the calibration gas 18 containing a controlled, very low concentration of the sample can be obtained.

In order to obtain the calibration gas 18 containing the very low concentration of the sample at 1 ppb level or below, it is important that the generation of the impurities at the two-stage gas dilution portion should be reduced. Further, in order to improve the concentration accuracy and also to cause the concentration of the very low-concentration calibration gas 18 to be changed in a real time manner in response to the change of the set value of the concentration by the control of the flow controllers 27, 25, 28 and 26, it is important that the equilibrium between the adsorption and desorption of the sample relative to the inner surface of the tube should be established rapidly. To achieve this, the two-stage gas dilution system is constituted by tubes subjected to an inner surface treatment, or glass tubes, connected together entirely by welding so as to provide a compact construction of the two-stage dilution portion and also to reduce the dead zones, and further the temperature of the tube is maintained by heating means 31 at a temperature 20 to 50 °C higher than the boiling temperature of the sample 7. Further, the flow control devices, such as the valves and the flow controllers, which would form sources of impurities are all mounted on the tubes of the branches, and are not mounted on the main tube 12 through which the standard or calibration gas flows.

Using this two-stage gas dilution system of Fig. 4, there can be prepared calibration gases containing the vapor of the liquid sample in a wide concentration range from a ppb level to a ppm level.

Fig. 5 shows an installation in which a highly-purified gas used in clean rooms is supplied in a branched manner from a highly-purified gas supplying system directly to a gas dilution system similar to that shown in Fig. 4. Line gas 32 is taken from the branch via a valve 33, and is controlled in pressure and flow rate by a gas regulator 34 and flow controllers 4, 25 and 26. The method of preparing a high-concentration standard gas 10 and the two-stage gas dilution method are the same as according to Fig. 4.

In this embodiment, the gas used in clean rooms can be directly introduced into the apparatus, and therefore additional facilities for installing gas cylinders are unnecessary, and advantageously the gas can be supplied for a long period of time, and time and labor for the exchange of the gas cylinders are not required. In this embodiment, instead of the line gas, a cylinder gas may be used, in which case only one gas cylinder is needed, which is more convenient as compared with the apparatus of Fig. 4.

Fig. 6 illustrates a method in which the calibration of the concentration of the high-concentration standard gas 10, obtained by passing the gas 1 through the high-concentration standard gas preparing device of Fig. 4, is carried out not by a microbalance, but by an analysis device 38 such as a gas chromatograph. After the gas 10 is branched, this branch gas 35 is introduced into the analysis device 38 for a pre-determined period of time by opening and closing a valve 36. The thus introduced gas is diluted with carrier gas 37, and is subjected to chromatography. The analysis device 38 has beforehand effected the concentration calibration with respect to the sample 7, and therefore the concentration can be known immediately from the data obtained.

Referring to the advantages of this embodiment, it does not take a long period of time to calibrate the concentration of the high-concentration standard gas 10 (the time required for this calibration is about 10 h), and the sample vessel 8 does not need to be taken out from the mixing chamber 6 each time the calibration is effected, and the concentration of the high-concentration standard gas 10 can be always monitored continuously.

Fig. 7 shows a further preferred embodiment of the invention in which using the apparatus shown in Fig. 4, the calibration of an analysis device (e.g. APIMS spectrometer) for analyzing a very low concentration of impurities in gas is carried out.

The gas dilution system of Fig. 4 is connected directly to the analysis device 41 for analyzing a very low concentration of impurities in gas, and the very low-concentration calibration gas 18 is introduced into the analysis device 41, so that the spectra can be observed.

By controlling the flow controllers 25, 26, 27 and 28, the concentration is set to a desired value, and the ion intensity of the sample is measured with respect to each of the concentrations, and the calibration curve representative of the relation between the concentration and the ion intensity can be measured.

In this embodiment, the flow controllers 4, 25, 26, 27 and 28 are controlled by a flow controller-controlling unit 39 and a computer system 40. More specifically, when the set concentrations are input into the computer system 40, the set values of the flow rates L1 to L5 (here, L1 is constant) are automatically calculated, and a flow rate-setting data signal 42 is fed to the controlling unit 39. The controlling unit 39 sends set flow rate signals 43 to the respective flow controllers, and reads flow rate data signals 44, fed respectively from the flow controllers, so as to confirm that the flow rates have been properly set respectively in the flow controllers. Thereafter, the controlling unit 39 sends a confirmation signal 45 to the computer system 40. Upon receipt of this confirmation signal 45, the computer system 40 sends a data input-starting signal 46 to the analysis device 41, and then receives a data signal 47 from the analysis device 41 and processes this data signal to prepare the spectra.

As in the embodiment of Fig. 3, there is the great advantage that the calibration curve of the analysis device for analyzing the very low concentration of impurities in the gas containing the liquid sample 7 can be automatically produced. And besides, the computer system 40 can be used not only to control the flow controllers but also to control the analysis device for analyzing the very low concentration of impurities in the gas, and therefore the cost can be reduced.

The apparatus of Fig. 7 was actually prepared, and high-purity nitrogen gas was used as the gases 1, 20 and 22; and the background was measured using APIMS. Data of these measurements are shown in Fig. 8. The two-stage gas dilution system was made with tubes of SUS316L-EP, and the main tube 12 was shortened to 30 cm, thereby reducing the background of the water to 2 ppb.

A calibration nitrogen gas containing a very low concentration of water at a ppb level was prepared using the apparatus of Fig. 7, and the relation between the ion intensity and the water concentration (calibration curve) was measured. Results of these measurements are shown in Fig. 9.

The calibration curve is satisfactorily linear, and this indicates that the dilution was carried out properly. Further, the time required for establishing the equilibrium between the adsorption and desorption was within several tens of seconds. Therefore, a real time analysis could be made.

## Claims

1. Method for gas analysis, comprising the following steps:
   - putting in the concentration of a sample gas in a calibration gas (18) into a computer system (40) to permit the system to calculate the flow rate (L3) of a zero gas (20) to be mixed with sample gas,
   - supplying the zero gas (20) from a zero gas source (19) to a main tube (12),
   - supplying the sample gas from a sample gas generating device (6) to the main tube (12) to mix the sample gas with the zero gas (20) in the main tube (12),

- controlling the flow rate of the zero gas (20) through flow control means (25) of the zero gas (20) by a set flow rate signal (43) of the calculated flow rate (L3) fed from the computer system (40) to thereby generate the calibration gas (18),
- supplying the calibration gas (18) to an analysis device (41), and
- feeding a data input-starting signal (46) from the computer system (40) to the analysis device (41), and feeding a data signal (47) from the analysis device (41) to the computer system (40).

2. The method according to claim 1, wherein the analysis device (41) comprises an atmospheric pressure ionization mass spectrometer.

3. The method according to claim 1 and/or 2, wherein the computer system (40) serves to perform processing of the data signals (47).

4. The method according to one or several of claims 1 to 3, wherein the computer system (40) receives a flow rate data signal (44) fed from the flow control means (25) to control the flow control means (25) in feeding the set flow rate signal (43) to the computer system (40).

5. The method according to one or several of claims 1 to 4, wherein
- a confirmation signal (45) is sent from a controlling unit (39) to the computer system (40), the controlling unit (39) receiving flow rate-setting signals (42) from the computer system (40) and putting out the set flow rate signals (43) and receiving the flow rate data signals (44) from the flow control means (25), and
- the computer system (40) sends a data input-starting signal (46) to the analysis device (41) and then receives data signals (47) from the analysis device (41) and processes the data signals (47) to generate the analysis data, preferably in the form of spectra.

6. The method according to one or several of claims 1 to 5, wherein the calibration gas (18) is prepared by the following steps:
- supplying a first zero gas (1) from a first zero gas source (2, 3, 4; 32, 33, 34) with a controlled flow rate (L1),
- supplying a sample gas from a sample gas source (7) with a controlled flow rate,
- mixing the sample gas with the first zero gas (1) in a mixing chamber (6) thus forming a first standard gas (10) containing the sample gas in a predetermined concentration,
- discarding a part of the first standard gas (10) at a first branch (13) with a controlled flow rate (L2), and
- mixing the remaining first standard gas at a second branch (14) downstream of the first branch (13) with a second zero gas (20) from a second zero gas source (19, 23, 25; 32, 33, 34) with a controlled flow rate (L3) to obtain a diluted, second standard gas,
wherein the sample gas is supplied by vaporizing a liquid sample (7) as sample gas source with a controlled evaporation rate,
and wherein the diluted standard gases are prevented from coming into contact with any parts such as valves and flow controllers,
to obtain a calibration gas (18) containing the sample gas in a very low concentration.

7. The method according to claim 6, wherein a part of the second standard gas is discarded at a third branch (15) with a predetermined flow rate (L4), and
the remaining diluted standard gas is mixed at a fourth branch (16) downstream of the third branch (15) with a third zero gas (22) from a third zero gas source (21, 24, 26; 32, 33, 34) with a controlled flow rate (L5).

8. The method according to claim 6 and/or 7, characterized by one or a combination of the following measures:
- the amount of vaporized liquid sample (7) is measured with a microbalance or with an analysis device (38);
- the first and second and optionally the third zero gases are provided from one gas source line (32, 33, 34);
- the zero gas (1, 20, 22) is purified directly before mixing with the sample gas or standard gas, respectively;

- the main tube (12) is maintained at a temperature 20 to 50 °C higher than the boiling point of the liquid sample.

9. Apparatus for gas analysis comprising
- a main tube (12) connected at its one end to an analysis device (41) for forming a calibration gas (18) from a zero gas (20) and a sample gas,
- zero gas source means (19, 23, 25) for supplying zero gas to the main tube (12), the gas source means comprising flow control means (25) for controlling the flow rate (L3) of the zero gas (20),
- a sample gas generating device (6) for supplying the sample gas to the main tube (12), and
- a computer system (40) for calculating the flow rate (L3) of the zero gas (20) for a concentration of the sample gas in the calibration gas, which concentration is put in into the computer system (40), feeding a set flow rate signal (43) of the calculated flow rate (L3) to the flow control means (25) to control the flow rate (L3), feeding a data input-starting signal (46) to the analysis device (41), and receiving a data signal (47) from the analysis device (41).

10. The apparatus according to claim 9, wherein the analysis device (41) comprises an atmospheric pressure ionization mass spectrometer.

11. The apparatus according to claim 9 and/or 10, wherein the computer system (40) serves to perform processing of the data signals (47).

12. The apparatus according to one or several of claims 9 to 11, wherein the computer system (40) is connected to a controlling unit (39) which receives a flow-rate setting signal (42) from the computer system (40), feeds a set flow rate signal (43) to the flow control means (25), receives a flow rate control signal (44) from the flow control means (25), controls the flow control means (25) so as to confirm the flow rate (L3) of the flow control means (25), and feeds a confirmation signal (45) to the computer system (40).

13. The apparatus according to one or several of claims 9 to 12, wherein the computer system (40) receives a flow rate data signal (44) fed from the flow control means (25) to control the flow control means (25) through the set flow rate signals (43).

14. The apparatus according to one or several of claims 9 to 13, which comprises a device for preparing the calibration gas, the device comprising
- a first and a second zero gas source (2, 3, 4; 19, 23, 25; 32, 33, 34) for supplying a first zero gas (1) and a second zero gas (20), comprising means (4, 25) for controlling the flow rates (L1, L3) of the first or second zero gases (1, 20), respectively,
- a sample gas source (7) for supplying a sample gas, comprising means for controlling the flow rate of the sample gas,
- a mixing chamber (6) for mixing the sample gas with the first zero gas (1), thus forming a first standard gas (10), and
- a main tube (12) connected with the mixing chamber (6) and having
(a) a first branch (13) comprising controlling means (27) for discarding a part of the first standard gas (10) with a controlled flow rate (L2),
(b) a second branch (14) provided downstream of the first branch (13), at which the remaining first standard gas is mixed with the second zero gas (20) with a controlled flow rate (L3) for obtaining a diluted, second standard gas,
wherein the mixing chamber (6) contains a sample vessel (8) in which a liquid sample (7) may be vaporized for supplying the sample gas, and the means for controlling the flow rate of the sample gas are means for controlling the evaporation rate of the liquid sample from the sample vessel (8), the main tube (12) not being provided with any parts such as valves and flow controllers.

15. The apparatus according to claim 14, wherein the main tube (12) is provided with
(a) a third branch (15) comprising controlling means (28) for discarding a part of the diluted second standard gas with a predetermined flow rate (L4), and

14

(b) a fourth branch (16) provided downstream of the third branch (15), at which the remaining diluted second standard gas is mixed with a third zero gas (22) supplied from a third zero gas source (21, 24, 26; 32, 33, 34) comprising means (26) for controlling the flow rate (L5) of the third zero gas (22).

16. The apparatus according to claim 14 and/or 15, characterized by one or a combination of the following features:
- the sample vessel (8) comprises a variable capillary aperture, preferably of 0.1 to 5 mm, for adjusting the evaporation rate of the liquid sample;
- the sample vessel (8) comprises a planar or curved upper surface which has no projection;
- the tubes are made of electropolished stainless steel or glass, the steel tubes being connected together entirely by welding;
- control means (39, 40) are provided to control the flow rates of the flow controlling means (4, 25, 26, 27, 28) and the concentration of the calibration gas automatically;
- the mixing chamber (6) is provided with temperature control means (9) for controlling the evaporation rate of the liquid sample from the sample vessel (8);
- adsorption reduction means (31) are provided at the main tube (12) for reducing the gas adsorption, preferably means for maintaining the main tube (12) at a temperature 20 to 50 °C higher than the boiling point of the liquid sample (7);
- impurity removal devices (5, 29, 30) are provided in the zero gas supply lines directly before the mixing chamber (6) or at the branches (14, 16), respectively.

17. Application of the method according to claims 1 to 8 and of the apparatus according to claims 9 to 16 to the analysis of impurities present in a gas in a very low concentration.

FIG. 1

# F I G. 2

# FIG. 3

# F I G. 4

# FIG. 5

EXHAUST

EXHAUST

HIGHLY PURIFIED GAS

EP 0 684 470 A2

# FIG. 6

21

# FIG. 7

# F I G. 8

# F I G. 9